# EUROPÄISCHE PATENTSCHRIFT

(11) **EP 4 184 155 B1**
(45) Veröffentlichungstag und Bekanntmachung des Hinweises auf die Patenterteilung: **09.09.2026**
(21) Anmeldenummer: 22195593.3
(22) Anmeldetag: 14.09.2022
(51) Int. Cl.: G01N 27/02, G01N 27/20, G01N 33/44

(54) **VERFAHREN ZUR ERMÜDUNGSMESSUNG EINES ELASTOMERPRODUKTS**
METHOD FOR FATIGUE MEASUREMENT OF AN ELASTOMERIC PRODUCT
PROCÉDÉ DE MESURE DE LA FATIGUE D'UN PRODUIT ÉLASTOMÈRE

(30) Priorität: 18.11.2021 DE 102021212990
(43) Veröffentlichungstag der Anmeldung: 24.05.2023
(73) Patentinhaber: ContiTech Deutschland GmbH, 30175 Hannover (DE)
(72) Erfinder: Reck, Siegfried, 30165 Hannover (DE)
(74) Vertreter: Preusser, Andrea

(56) Entgegenhaltungen:
- WO-A1-2020/020534
- DE-A1- 10 008 481
- GB-A- 2 187 291
- US-A1- 2006 164 106

## Beschreibung

Die vorliegende Erfindung betrifft ein Verfahren zur Ermüdungsmessung eines Elastomerprodukts, ein Ermüdungsmesssystem für Elastomerprodukte sowie einen Ermüdungssensor.

In vielen Bereichen des Maschinen- und Fahrzeugbaus werden Bauteile mit elastomeren Funktionselementen eingesetzt, wie z. B. Luftfedern, MeGi-Federn, Riemen, Transportbänder, Schläuche etc. Aufgrund von Alterung und dynamischer mechanischer Belastung ermüden elastomere Werkstoffe. Für den Einsatz bei der zustandsabhängigen Wartung werden Systeme und Verfahren entwickelt, die den Alterungs- bzw. den Ermüdungszustand der elastomeren Materialien zerstörungsfrei ermitteln. Sie beruhen auf der Erkenntnis, dass sich die Permittivität elastomerer Werkstoffe verändert, wenn sie ermüden, z. B. dadurch, dass ihre Makromoleküle und bzw. oder deren Quervernetzungen zerbrechen oder dass sich ihre Füllstoffmatrix destabilisiert.

Die EP 2 375 099 A2 beschreibt einen resonanten Sensor aus einer in sich geschlossenen, leitfähigen Struktur, deren Resonanzfrequenz von der Permittivität ihrer Umgebung abhängig ist. Eingebettet in das zu überwachende Bauteil ändert sich die Resonanzfrequenz der Leiterstruktur mit dem Ermüdungszustand des Elastomers.

Die DE 10 2014 213 969 A1 beschreibt einen Dipol aus leitfähigem Elastomer, dessen dielektrische Eigenschaften mit denen des ihn umgebenden Elastomers so abgestimmt sind, dass der Dipol auf eine eintreffende elektromagnetische Prüfwelle mit einem definierten resonanten Signal antwortet, das dem Ermüdungszustand des umgebenden Elastomers entspricht.

Nachteilig ist sowohl hinsichtlich der leitfähigen Struktur der EP 2 375 099 A2 als auch hinsichtlich des Dipols der DE 10 2014 213 969 A1, dass diese zusätzliche Elemente darstellen, die in das zu überwachende Bauteil integriert werden müssen, um ihre Funktion zu erfüllen. Bei zu großen Unterschieden bezüglich des E-Moduls können die Überwachungselemente die strukturelle Integrität des Bauteils gefährden. Besonders nachteilig sind metallische Leiter, wenn sie in dynamisch belasteten Regionen des zu überwachenden Bauteils integriert werden, denn sie widerstehen nur eine vergleichsweise geringe Anzahl an Lastwechseln.

Außerdem muss sich das Elastomer des Dipols der DE 10 2014 213 969 A1 bezüglich seiner dielektrischen Eigenschaften von seiner Umgebung unterscheiden, damit der Dipol für den Sende-Empfänger erkennbar ist. Die Auswahl und Verwaltung der Werkstoffpaarungen verursachen Aufwand und Kosten.

Die Lösungen der EP 2 375 099 A2 und der DE 10 2014 213 969 A1 beruhen ferner darauf, dass sich bei der Ermüdung des Elastomers dessen Dielektrizitätskonstante verändert. Durch die Auswertung der Resonanzfrequenz als Prüfkriterium wird in beiden Fällen lediglich der Realteil der komplexen Dielektrizitätskonstanten betrachtet, so dass diese Methoden wegen ihrer geringen Sensitivität lediglich eine vage Vorhersage der Restlebensdauer ermöglichen.

Die US 2006/164106 A1 offenbart ein Verfahren zur Bestimmung des Feuchtigkeitsgehalts in Elastomermaterialien durch kapazitive Messung. Dabei wird die Kapazität des Elastomers ermittelt und mit der Kapazität einer Referenzprobe verglichen, sodass in jedem Fall zwei Messungen getätigt und gegenübergestellt werden müssen.

Eine Aufgabe der vorliegenden Erfindung ist es, die Möglichkeiten zur Beurteilung der Materialermüdung eines Elastomerprodukts bzw. dessen Elastomerkörper zu verbessern. Insbesondere soll die Qualität der Aussage über den Materialzustand bzw. über die Materialermüdung verbessert werden. Dies soll insbesondere möglichst einfach, kostengünstig, bauraumsparend, energiesparend und bzw. oder flexibel anwendbar erfolgen. Zumindest soll eine Alternative zu den bekannten derartigen Möglichkeiten geschaffen werden.

Die Aufgabe wird erfindungsgemäß durch ein Verfahren sowie durch ein Ermüdungsmesssystem gemäß der unabhängigen Ansprüche gelöst. Vorteilhafte Weiterbildungen sind in den Unteransprüchen beschrieben.

Somit betrifft die vorliegende Erfindung ein Verfahren zur Ermüdungsmessung eines Elastomerprodukts, wobei ein Ermüdungssensor mit wenigstens einem kapazitiven Sensorelement derart auf einer Oberfläche eines Elastomerkörpers des Elastomerprodukts angeordnet ist, so dass die elektrischen Feldlinien des kapazitiven Sensorelements in den Elastomerkörper eindringen können, mit wenigstens den Schritten:
- Anregen des kapazitiven Sensorelements, vorzugsweise drahtlos,
- Erfassen sowohl des Realteils als auch des Imaginärteils der Impedanz des kapazitiven Sensorelements über einen Frequenzbereich;
- Bestimmen des Verlustwinkels der Impedanz über den Frequenzbereich;
- Vergleichen des frequenzabhängigen Verlustwinkels mit wenigstens einem vorbestimmten frequenzabhängigen Verlustwinkel eines bekannten Ermüdungszustands des Elastomerkörpers; und
- Bestimmen eines Grades der Ermüdung des Elastomerkörpers aus dem Ergebnis des Vergleichens.

Somit geht die vorliegende Erfindung von der Erkenntnis aus, dass die komplexe Permittivität eine empfindlichere Messgröße ist als deren Realteil oder deren Betrag. Erfindungsgemäß kann daher auf einer Oberfläche eines zu überwachenden elastomeren Bauteils ein kapazitives Sensorelement, beispielsweise in Form einer zweipoligen, interdigitalen Leiterstruktur, aufgebracht werden, so dass dessen elektrische Feldlinien in die Oberfläche des elastomeren Bauteils bzw. dessen Elastomerkörper eindringen können. Ein programmierbares Impedanz-Spektrometer oder dergleichen kann die Impedanz der sensorischen Leiterstruktur über den Frequenzbereich erfassen bzw. messen, indem das zu überwachende Elastomer bei elektrischer Anregung Raumladungs-Relaxation und Orientierungsrelaxation zeigt. Die elektrische Anregung kann drahtgebunden mittels einer elektrischen Wechselspannung oder drahtlos mittels eines elektrischen Wechselfeldes erfolgen, wobei letzteres die Umsetzung vereinfachen kann.

Als Maß für die Ermüdung des Elastomers wird erfindungsgemäß aus dem Verhältnis zwischen dem Imaginärteil und dem Realteil der messbaren Impedanz der sogenannte Verlustwinkel (tangens_delta) bestimmt. Über wenigstens eine oder vorzugsweise mehrere werkstoffspezifische oder bauteilspezifische Kennlinie bzw. Kennlinien kann dem ermittelten Verlustwinkel ein Grad der Ermüdung zugeordnet werden.

Ein entsprechendes erfindungsgemäßes Verfahren bzw. Ermüdungsmesssystem eignet sich sowohl für wiederkehrende Inspektionen als auch zur dauerhaften Überwachung von elastomeren Bauteilen. Aufgrund seiner erhöhten Sensitivität kann das entsprechende Sensorelement in weniger stark belasteten Bereichen des zu überwachenden Bauteils appliziert werden. Die intelligente Elektronik des erfindungsgemäßen Ermüdungsmesssystems kann universell einsetzbar sein und sich bezüglich des Frequenzganges der Messung und der Bewertung der Messergebnisse per Software an verschiedene elastomere Werkstoffe anpassen lassen.

Das entsprechende erfindungsgemäße Verfahren bzw. Ermüdungsmesssystem kann auf jegliche Elastomere bzw. elastomere Produkte wie beispielsweise auf Luftfedern, Riemen, Gummi-Metall-Lager, Schläuche und dergleichen angewendet werden.

Gemäß einem Aspekt der Erfindung erfolgt das Vergleichen des frequenzabhängigen Verlustwinkels mit einer Mehrzahl von vorbestimmten frequenzabhängigen Verlustwinkeln verschiedener bekannter Ermüdungszustände des Elastomerkörpers erfolgt. Dies kann die Qualität der Schlussfolgerung erhöhen, da mehrere bekannte Kennlinien betrachtet werden und somit die gemessene Kennlinien besser einer der bekannten Kennlinien zugeordnet werden kann bzw. der verbleibende Unterschied verringert werden kann.

Gemäß einem weiteren Aspekt der Erfindung liegt der Frequenzbereich zwischen 10 kHz und 10 MHz. Dies kann eine wirkungsvolle Umsetzung ermöglichen, da ein repräsentativer Frequenzbereich angeregt und gemessen werden kann. Dies kann die Genauigkeit der gemessene Kennlinie erhöhen.

Gemäß einem weiteren Aspekt der Erfindung weist das kapazitive Sensorelement eine Mehrzahl erster Stege und eine korrespondierende Mehrzahl zweiter Stege auf, welche kammartig zueinander angeordnet sind. Dies kann die Messempfindlichkeit erhöhen, da die wirksame Fläche des kapazitiven Sensorelements vergrößert werden kann.

Gemäß einem weiteren Aspekt der Erfindung sind die Stege auf einem Trägerelement, vorzugsweise durch Drucken, angeordnet. Dies kann die Handhabung des kapazitiven Sensorelement vereinfachen. Die Umsetzung durch Drucken kann die Herstellungskosten verringern und bzw. oder besonders feine Strukturen bzw. Stege ermöglichen, was ebenfalls die wirksame Fläche des kapazitiven Sensorelements vergrößern kann.

Gemäß einem weiteren Aspekt der Erfindung ist das Trägerelement mit seiner den Stegen abgewandten Seite stoffschlüssig, vorzugsweise durch Kleben, auf dem Elastomerkörper des Elastomerprodukts angeordnet. Dies kann eine sichere und haltbare Anordnung ermöglichen.

Die vorliegende Erfindung betrifft auch ein Ermüdungsmesssystem für Elastomerprodukte mit einem Ermüdungssensor mit wenigstens einem kapazitiven Sensorelement, welches ausgebildet ist, auf einer Oberfläche eines Elastomerkörpers des Elastomerprodukts derart angeordnet zu werden, so dass die elektrischen Feldlinien des kapazitiven Sensorelements in den Elastomerkörper eindringen können, mit einem Spektrometer, welches ausgebildet ist, das kapazitive Sensorelement, vorzugsweise drahtlos, anzuregen und sowohl den Realteil als auch den Imaginärteil der Impedanz des kapazitiven Sensorelements über einen Frequenzbereich zu erfassen, wobei das Spektrometer ferner ausgebildet ist, den Verlustwinkel der Impedanz über den Frequenzbereich zu bestimmen, und mit einer Recheneinheit, welche ausgebildet ist, den frequenzabhängigen Verlustwinkel mit wenigstens einem vorbestimmten frequenzabhängigen Verlustwinkel eines bekannten Ermüdungszustands des Elastomerkörpers zu vergleichen und aus dem Ergebnis des Vergleichens einen Grad der Ermüdung des Elastomerkörpers zu bestimmen, wobei alternativ zum Spektrometer die Recheneinheit ferner ausgebildet ist, den Verlustwinkel der Impedanz über den Frequenzbereich zu bestimmen, und bzw. oder wobei das Anregen des kapazitiven Sensorelements alternativ zum Spektrometer, vorzugsweise mittels einer Sendeeinheit, erfolgen kann.

Hierdurch kann ein Ermüdungsmesssystem geschaffen werden, um das zuvor beschriebene Verfahren umsetzen zu können.

Gemäß einem Aspekt der Erfindung ist die Recheneinheit ausgebildet, den frequenzabhängigen Verlustwinkel mit einer Mehrzahl von vorbestimmten frequenzabhängigen Verlustwinkeln verschiedener bekannter Ermüdungszustände des Elastomerkörpers zu vergleichen. Hierdurch können die entsprechenden Aspekte des erfindungsgemäßen Verfahrens auch seitens des erfindungsgemäßen Ermüdungsmesssystems umgesetzt und genutzt werden.

Gemäß einem weiteren Aspekt der Erfindung liegt der Frequenzbereich zwischen 10 kHz und 10 MHz. Hierdurch können die entsprechenden Aspekte des erfindungsgemäßen Verfahrens auch seitens des erfindungsgemäßen Ermüdungsmesssystems umgesetzt und genutzt werden.

Gemäß einem weiteren Aspekt der Erfindung weist das Ermüdungsmesssystem eine Speichereinheit auf, welche ausgebildet ist, die vorbestimmte frequenzabhängige Impedanz eines bekannten Ermüdungszustands des Elastomerkörpers, vorzugsweise eine Mehrzahl von vorbestimmten frequenzabhängigen Impedanzen verschiedener bekannter Ermüdungszustände des Elastomerkörpers, zu speichern und der Recheneinheit zur Verfügung zu stellen. Hierdurch können die entsprechenden Informationen der Recheneinheit zur Verfügung gestellt werden.

Gemäß einem weiteren Aspekt der Erfindung weist das kapazitive Sensorelement eine Mehrzahl erster Stege und eine korrespondierende Mehrzahl zweiter Stege auf, welche kammartig zueinander angeordnet sind. Hierdurch können die entsprechenden Aspekte des erfindungsgemäßen Verfahrens auch seitens des erfindungsgemäßen Ermüdungsmesssystems umgesetzt und genutzt werden.

Gemäß einem weiteren Aspekt der Erfindung sind die Stege auf einem Trägerelement, vorzugsweise durch Drucken, angeordnet. Hierdurch können die entsprechenden Aspekte des erfindungsgemäßen Verfahrens auch seitens des erfindungsgemäßen Ermüdungsmesssystems umgesetzt und genutzt werden.

Gemäß einem weiteren Aspekt der Erfindung ist das Trägerelement mit seiner den Stegen abgewandten Seite stoffschlüssig, vorzugsweise durch Kleben, auf dem Elastomerkörper des Elastomerprodukts angeordnet. Hierdurch können die entsprechenden Aspekte des erfindungsgemäßen Verfahrens auch seitens des erfindungsgemäßen Ermüdungsmesssystems umgesetzt und genutzt werden.

Die vorliegende Offenbarung beschreibt auch einen Ermüdungssensor zur Verwendung in einem Verfahren wie zuvor beschrieben und bzw. oder in einem Ermüdungsmesssystem wie zuvor beschrieben, wobei der Ermüdungssensor wenigstens ein kapazitives Sensorelement aufweist, welches ausgebildet ist, derart auf einer Oberfläche eines Elastomerkörpers eines Elastomerprodukts angeordnet zu werden, so dass die elektrischen Feldlinien des kapazitiven Sensorelements in den Elastomerkörper eindringen können. Hierdurch kann ein Ermüdungssensor geschaffen werden, um das zuvor beschriebene Verfahren und bzw. oder das zuvor beschriebene Ermüdungsmesssystem umsetzen zu können.

Gemäß einem Aspekt des beschriebenen Ermüdungssensors ist das kapazitive Sensorelement ausgebildet, drahtlos angeregt zu werden. Hierdurch können die entsprechenden Aspekte des erfindungsgemäßen Verfahrens bzw. des erfindungsgemäßen Ermüdungsmesssystems auch seitens des beschriebenen Ermüdungssensors umgesetzt und genutzt werden.

Ein Ausführungsbeispiel und weitere Vorteile der Erfindung werden nachstehend im Zusammenhang mit den folgenden Figuren erläutert. Darin zeigt:
- Fig. 1: eine schematische Draufsicht auf einen Ermüdungssensor eines erfindungsgemäßen Ermüdungsmesssystems;
- Fig. 2: eine seitliche Schnittansicht des Ermüdungssensors bei Verwendung auf einem Elastomerprodukt;
- Fig. 3: eine Draufsicht auf das erfindungsgemäße Ermüdungsmesssystem;
- Fig. 4: zwei Messdiagramme des erfindungsgemäßen Ermüdungsmesssystems; und
- Fig. 5: ein Ablaufdiagramm eines erfindungsgemäßen Verfahrens zur Ermüdungsmessung des Elastomerprodukts.

Die Beschreibung der o.g. Figuren erfolgt in kartesischen Koordinaten mit einer Längsrichtung X, einer zur Längsrichtung X senkrecht ausgerichteten Querrichtung Y sowie einer sowohl zur Längsrichtung X als auch zur Querrichtung Y senkrecht ausgerichteten vertikalen Richtung Z. Die Längsrichtung X kann auch als Tiefe X, die Querrichtung Y auch als Breite Y und die vertikale Richtung Z auch als Höhe Z bezeichnet werden. Die Längsrichtung X und die Querrichtung Y bilden gemeinsam die Horizontale, X, Y, welche auch als horizontale Ebene X, Y bezeichnet werden kann. Die Längsrichtung X, die Querrichtung Y und die vertikale Richtung Z können gemeinsam auch als Raumrichtungen X, Y, Z bzw. als kartesische Raumrichtungen X, Y, Z bezeichnet werden.

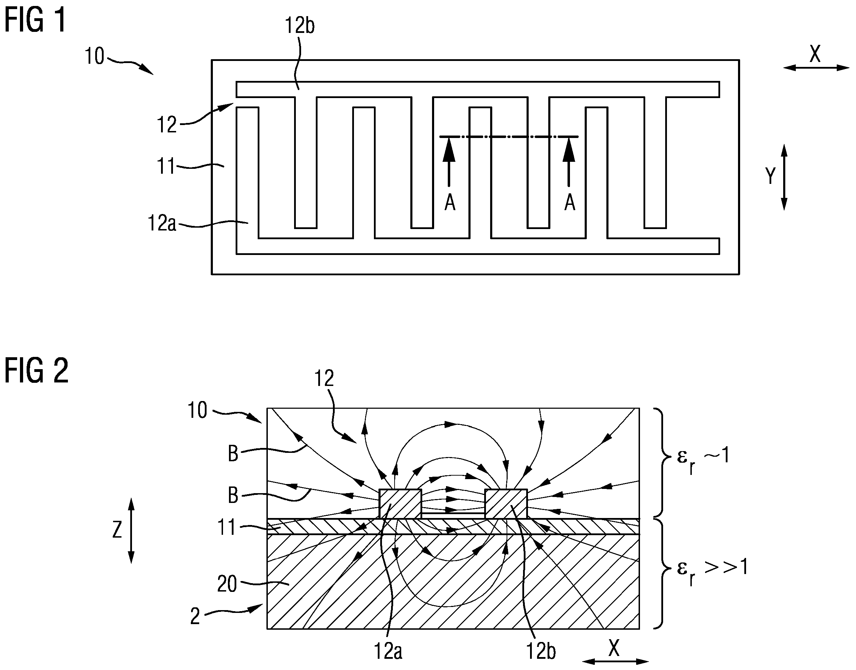

Figur 1 zeigt eine schematische Draufsicht auf einen Ermüdungssensor 10 eines erfindungsgemäßen Ermüdungsmesssystems 1. Fig. 2 zeigt eine seitliche Schnittansicht A-A des Ermüdungssensors 10 bei Verwendung auf einem Elastomerprodukt 2.

Der Ermüdungssensor 10 weist ein kapazitives Sensorelement 12 auf, welches auch als interdigitale Leiterstruktur 12 bezeichnet werden kann. Das kapazitive Sensorelement 12 weist eine erste Leiterbahn12a in Form kammartiger erster Stege 12a und eine korrespondierend ausgebildete und angeordnete zweite Leiterbahn12b in Form kammartiger zweiter Stege 12b auf, welches gemeinsam eine im Wesentlichen rechteckige Anordnung bilden, siehe Fig. 1. Das kapazitive Sensorelement 12 ist metallisch mittels Drucken auf einer Seite eines Trägerelements 11 in Form einer nicht-elektrisch leitfähigen dünnen Trägerfolie 11 aufgebracht. Die Trägerfolie 11 ist mittels Kleben auf einer Oberfläche eines Elastomerkörpers 20 des Elastomerprodukts 2 als zu überwachendes Elastomerprodukt 2 aufgebracht, welches beispielsweise eine Luftfeder, ein Riemen, ein Gummi-Metall-Lager, ein Schlauch oder dergleichen sein kann.

Dabei ist der Ermüdungssensor 10 bzw. dessen kapazitives Sensorelement 12 derart ausgebildet, dass bei elektrischer Anregung des kapazitiven Sensorelements 12 mittels einer Wechselspannung bzw. mittels eines Wechselfeldes die elektrischen Feldlinien B des kapazitiven Sensorelements 12 in den Elastomerkörper 20 des Elastomerprodukts 2 eindringen, siehe Fig. 2. Mit anderen Worten bilden die direkt benachbarten Stege 12a, 12b des kapazitiven Sensorelements 12 eine elektrische Kapazität. Wenn die Stege 12a, 12b aufgrund der Anregung auf unterschiedlichem Potenzial liegen, bildet sich zwischen ihnen ein elektrisches Feld mit den in der Fig. 2 dargestellten elektrischen Feldlinien B aus.

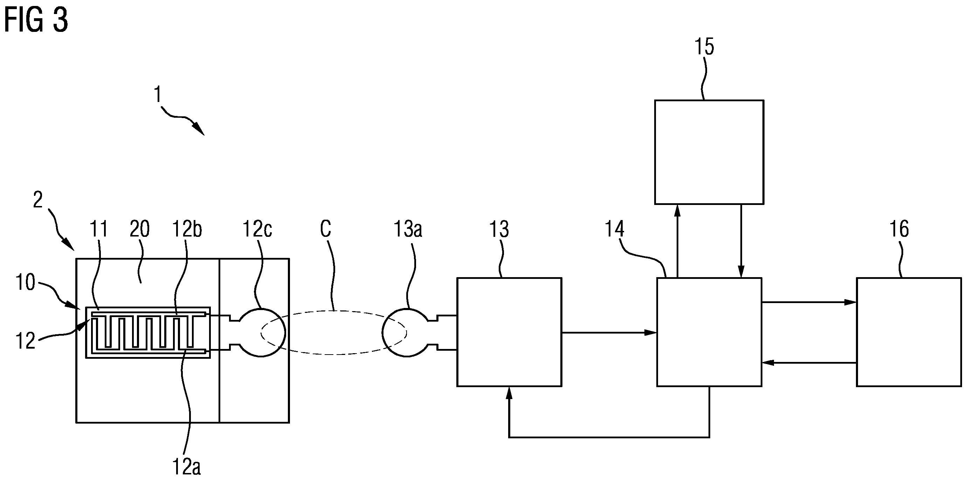

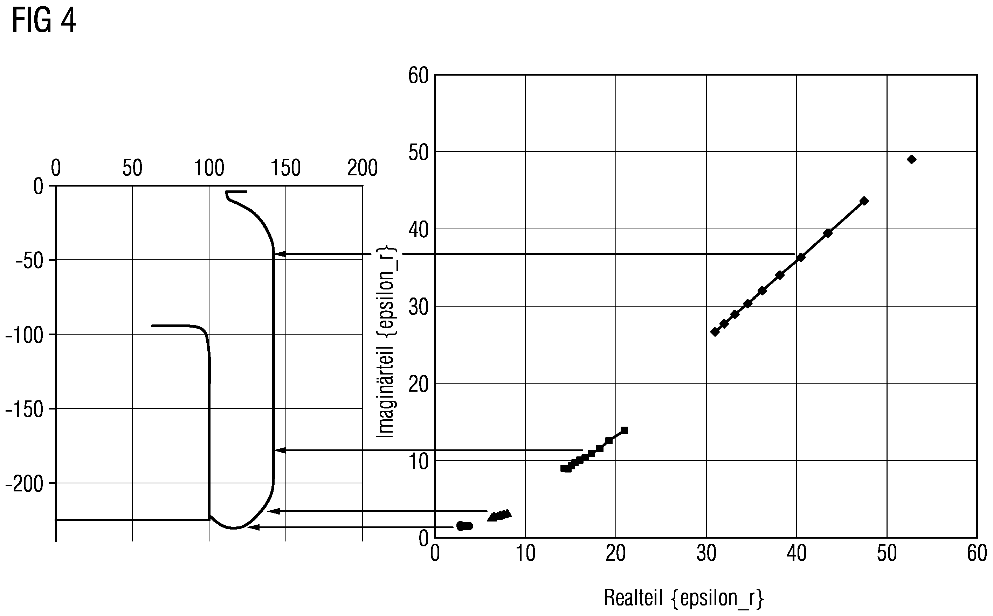

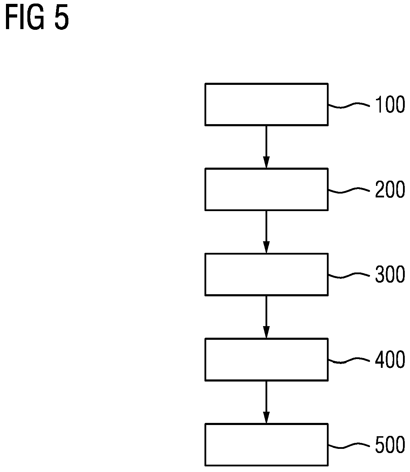

Fig. 3 zeigt eine Draufsicht auf das erfindungsgemäße Ermüdungsmesssystem 1. Fig. 4 zeigt zwei Messdiagramme des erfindungsgemäßen Ermüdungsmesssystems 1. Neben dem zuvor beschriebenen Ermüdungssensor 10 umfasst das Ermüdungsmesssystem 1 ein Spektrometer 13, eine Recheneinheit 14 mit einer Speichereinheit 15 als Festwertspeicher 15 sowie eine Schnittstelle 16. Entsprechend kann mittels des erfindungsgemäßen Ermüdungsmesssystem 1 ein erfindungsgemäßen Verfahren zur Ermüdungsmessung des Elastomerprodukts 2 gemäß des Ablaufdiagramm der Fig. 5 wie folgt ausgeführt werden:
In einem ersten Schritt 100 erfolgt ein Anregen des kapazitiven Sensorelements 12, was seitens des Spektrometer 13 insbesondere mittels einer elektrischen drahtlosen Anregung C in Form eines elektrischen Wechselfeldes C erfolgen kann. Hierbei wird seitens des Spektrometers 13 mittels einer Leiterschleife 13a das elektrische Wechselfeld C erzeugt und seitens des kapazitiven Sensorelements 12 mittels einer Leiterschleife 12c empfangen.

In einem zweiten Schritt 200 erfolgt mittels der Leiterschleife 13a des Spektrometers 13 ein Erfassen sowohl des Realteils als auch des Imaginärteils der Impedanz des kapazitiven Sensorelements 12 über einen Frequenzbereich, welcher vorzugsweise zwischen 10 kHz und 10 MHz liegt.

Seitens der Recheneinheit 14 erfolgt nun in einem dritten Schritt 300 ein Bestimmen des Verlustwinkels der Impedanz über den Frequenzbereich.

Ferner seitens der Recheneinheit 14 erfolgt in einem vierten Schritt 400 ein Vergleichen der frequenzabhängigen Impedanz mit wenigstens einer vorbestimmten frequenzabhängigen Impedanz eines bekannten Ermüdungszustands des Elastomerkörpers 20. Dies kann vorzugweise für mehrere vorbestimmte frequenzabhängige Impedanzen verschiedener bekannter Ermüdungszustände des Elastomerkörpers 20 erfolgen, um die Aussagekraft des Verfahrens zu erhöhen. In jedem Fall können die vorbestimmten Informationen seitens der Speichereinheit 15 der Recheneinheit 14 zur Verfügung gestellt werden.

Abschließend erfolgt in einem fünften Schritt 500 ein Bestimmen eines Grades der Ermüdung des Elastomerkörpers 20 aus dem Ergebnis des Vergleichens 400. Auch dies kann seitens der Recheneinheit 14 erfolgen. Der bestimmte Grad der Ermüdung des Elastomerkörpers 20 kann dann über die Schnittstelle 16, insbesondere drahtlos, nach außerhalb des erfindungsgemäßen Ermüdungsmesssystems 1 zur Verfügung gestellt werden.

Dabei liegt der Erfindung die Erkenntnis zugrunde, dass für die Messung der Impedanz nur der Teil des elektrischen Feldes B relevant ist, dessen Feldlinien B in dem zu überwachenden Elastomerkörper 20 verlaufen. Die Teilfelder oberhalb des kapazitiven Sensorelements 12 und direkt zwischen dessen Stegen 12a, 12b sind parasitär, siehe Fig. 2, weil sie die Gesamtkapazität der Anordnung erhöhen und dadurch deren Sensitivität verringern. Daher kann die Dielektrizitätskonstanten der entsprechenden Räume klein gehalten werden, um den Einfluss dieser parasitären Teilkapazitäten gering zu halten.

In dem Frequenzband von 10 kHz bis 10 MHz wird mittels des Impedanz-Spektrometers 13 dann den Real- und Imaginärteil der Impedanz der Leiterstrukturen 12a, 12b gemessen, dessen elektrisches Feld B in den zu überwachenden Elastomerkörper 20 eindringt. Hieraus wird dann seitens des Spektrometers 13 oder seitens der Recheneinheit 14 dessen Verlustwinkel berechnet.

Die Recheneinheit 14 ermittelt aus der frequenzabhängigen Kurve des Verlustwinkels eine Ausgleichsfunktion und vergleicht diese mit den Daten aus dem nichtflüchtigen Festwertspeicher 15, die zuvor im Neuzustand des Materials bzw. des Bauteils ermittelt wurden.

Aus den Abweichungen zwischen den Ausgleichskurven ermittelt die Recheneinheit 14 den Grad der Ermüdung des zu überwachenden Materials. Über die Datenschnittstelle 16 kann die Recheneinheit 14 die Parameter für die Messung variieren, um den Verlustwinkel für einzelne Frequenzen bzw. Frequenzbänder detailliert zu vermessen.

Das ermittelte Ergebnis für den Grad der Ermüdung kann mit Hilfe der Schnittstelle 16 an eine externe Einheit übertragen werden (z. B. ein IoT-Device). Außerdem kann die Schnittstelle 16 dazu genutzt werden, die material- bzw. produktspezifischen Daten in den Festwertspeicher 15 zu übertragen.

Die Fig. 4 zeigt den Imaginärteil des Verlustwinkels, der an verschieden stark belasteten Stellen einer Luftfeder als Beispiel eines Elastomerprodukts 2 gemessen wurde, aufgetragen über dem Realteil (gemessen über jeweils den gleichen Frequenzbereich). Je geringer die Distanz zwischen den Messpunkten und der Rollfalte der Luftfeder, desto stärker die Veränderungen bei der Steigung des Grafen. In der dynamisch besonders beanspruchten Rollfalte zeigen sich die stärksten Veränderungen des Verlustwinkels.

### Bezugszeichenliste (Teil der Beschreibung)

- A-A: Schnittansicht
- B: elektrische Feldlinien
- C: elektrische drahtlose Anregung; elektrisches Wechselfeld

- 1: Ermüdungsmesssystem
- 10: Ermüdungssensor
- 11: Trägerelement; Trägerfolie
- 12: kapazitives Sensorelement; interdigitale Leiterstruktur
- 12a: erste Leiterbahn bzw. erste Stege des kapazitiven Sensorelements 12
- 12b: zweite Leiterbahn bzw. zweite Stege des kapazitiven Sensorelements 12
- 12c: Leiterschleife
- 13: Spektrometer
- 13a: Leiterschleife
- 14: Recheneinheit
- 15: Speichereinheit; Festwertspeicher
- 16: Schnittstelle

- 2: Elastomerprodukt
- 20: Elastomerkörper

- 100: Anregen des kapazitiven Sensorelements 12
- 200: Erfassen sowohl des Realteils als auch des Imaginärteils der Impedanz des kapazitiven Sensorelements 12 über einen Frequenzbereich
- 300: Bestimmen des Verlustwinkels der Impedanz über den Frequenzbereich
- 400: Vergleichen der frequenzabhängigen Impedanz mit wenigstens einer vorbestimmten frequenzabhängigen Impedanz eines bekannten Ermüdungszustands des Elastomerkörpers 20
- 500: Bestimmen eines Grades der Ermüdung des Elastomerkörpers 20 aus dem Ergebnis des Vergleichens 400

## Patentansprüche

1. Verfahren zur Ermüdungsmessung eines Elastomerprodukts (2), wobei ein Ermüdungssensor (10) mit wenigstens einem kapazitiven Sensorelement (12) derart auf einer Oberfläche eines Elastomerkörpers (20) des Elastomerprodukts (2) angeordnet ist, so dass die elektrischen Feldlinien (B) des kapazitiven Sensorelements (12) in den Elastomerkörper (20) eindringen können,
mit wenigstens den Schritten:
• Anregen (100) des kapazitiven Sensorelements (12), vorzugsweise drahtlos,
• Erfassen (200) sowohl des Realteils als auch des Imaginärteils der Impedanz des kapazitiven Sensorelements (12) über einen Frequenzbereich;
**gekennzeichnet durch** folgende Schritte:
• Bestimmen (300) des Verlustwinkels der Impedanz über den Frequenzbereich;
• Vergleichen (400) des frequenzabhängigen Verlustwinkels mit wenigstens einem vorbestimmten frequenzabhängigen Verlustwinkel eines bekannten Ermüdungszustands des Elastomerkörpers (20); und
• Bestimmen (500) eines Grades der Ermüdung des Elastomerkörpers (20) aus dem Ergebnis des Vergleichens (400).

2. Verfahren nach Anspruch 1,
wobei das Vergleichen (400) des frequenzabhängigen Verlustwinkels mit einer Mehrzahl von vorbestimmten frequenzabhängigen Verlustwinkeln verschiedener bekannter Ermüdungszustände des Elastomerkörpers (20) erfolgt.

3. Verfahren nach Anspruch 1 oder 2,
wobei der Frequenzbereich zwischen 10 kHz und 10 MHz liegt.

4. Verfahren nach einem der Ansprüche 1 bis 3,
wobei das kapazitive Sensorelement (12) eine Mehrzahl erster Stege (12a) und eine korrespondierende Mehrzahl zweiter Stege (12b) aufweist, welche kammartig zueinander angeordnet sind.

5. Verfahren nach Anspruch 4,
wobei die Stege (12a, 12b) auf einem Trägerelement (11), vorzugsweise durch Drucken, angeordnet sind.

6. Verfahren nach Anspruch 5,
wobei das Trägerelement (11) mit seiner den Stegen (12a, 12b) abgewandten Seite stoffschlüssig, vorzugsweise durch Kleben, auf dem Elastomerkörper (20) des Elastomerprodukts (2) angeordnet ist.

7. Ermüdungsmesssystem (1) für Elastomerprodukte (2) mit einem Ermüdungssensor (10) mit wenigstens einem kapazitiven Sensorelement (12), welches ausgebildet ist, auf einer Oberfläche eines Elastomerkörpers (20) des Elastomerprodukts (2) derart angeordnet zu werden, so dass die elektrischen Feldlinien (B) des kapazitiven Sensorelements (12) in den Elastomerkörper (20) eindringen können, mit einem Spektrometer (13), welches ausgebildet ist, das kapazitive Sensorelement (12), vorzugsweise drahtlos, anzuregen, wobei das Anregen des kapazitiven Sensorelements (12) alternativ zum Spektrometer (13), vorzugsweise mittels einer Sendeeinheit, erfolgen kann, wobei das Spektrometer (13) ausgebildet ist, sowohl den Realteil als auch den Imaginärteil der Impedanz des kapazitiven Sensorelements (12) über einen Frequenzbereich zu erfassen,
**dadurch gekennzeichnet, dass** das Spektrometer (13) ferner ausgebildet ist, den Verlustwinkel der Impedanz über den Frequenzbereich zu bestimmen, wobei alternativ zum Spektrometer (13) die Recheneinheit (14) ferner ausgebildet ist, den Verlustwinkel der Impedanz über den Frequenzbereich zu bestimmen,
und mit einer Recheneinheit (14), welche ausgebildet ist, den frequenzabhängigen Verlustwinkel mit wenigstens einem vorbestimmten frequenzabhängigen Verlustwinkel eines bekannten Ermüdungszustands des Elastomerkörpers (20) zu vergleichen und aus dem Ergebnis des Vergleichens einen Grad der Ermüdung des Elastomerkörpers (20) zu bestimmen.

8. Ermüdungsmesssystem (1) nach Anspruch 7,
wobei die Recheneinheit (14) ausgebildet ist, den Verlustwinkel mit einer Mehrzahl von vorbestimmten frequenzabhängigen Verlustwinkeln verschiedener bekannter Ermüdungszustände des Elastomerkörpers (20) zu vergleichen.

9. Ermüdungsmesssystem (1) nach Anspruch 7 oder 8,
wobei der Frequenzbereich zwischen 10 kHz und 10 MHz liegt.

10. Ermüdungsmesssystem (1) nach einem der Ansprüche 7 bis 9,
mit einer Speichereinheit (15), welche ausgebildet ist, die vorbestimmte frequenzabhängige Impedanz eines bekannten Ermüdungszustands des Elastomerkörpers (20), vorzugsweise eine Mehrzahl von vorbestimmten frequenzabhängigen Impedanzen verschiedener bekannter Ermüdungszustände des Elastomerkörpers (20), zu speichern und der Recheneinheit (14) zur Verfügung zu stellen.

11. Ermüdungsmesssystem (1) nach einem der Ansprüche 7 bis 10,
wobei das kapazitive Sensorelement (12) eine Mehrzahl erster Stege (12a) und eine korrespondierende Mehrzahl zweiter Stege (12b) aufweist, welche kammartig zueinander angeordnet sind.

12. Ermüdungsmesssystem (1) nach Anspruch 11,
wobei die Stege (12a, 12b) auf einem Trägerelement (11), vorzugsweise durch Drucken, angeordnet sind.

13. Ermüdungsmesssystem (1) nach Anspruch 12,
wobei das Trägerelement (11) mit seiner den Stegen (12a, 12b) abgewandten Seite stoffschlüssig, vorzugsweise durch Kleben, auf dem Elastomerkörper (20) des Elastomerprodukts (2) angeordnet ist.

## Claims

1. A method for measuring fatigue of an elastomer product (2),
wherein a fatigue sensor (10) having at least one capacitive sensor element (12) is arranged on a surface of an elastomer body (20) of the elastomer product (2) such that the electric field lines (B) of the capacitive sensor element (12) can penetrate into the elastomer body (20),
comprising at least the following steps:
• stimulating (100) the capacitive sensor element (12), preferably wirelessly;
• detecting (200) both the real part and the imaginary part of the impedance of the capacitive sensor element (12) over a frequency range;
**characterized by** the following steps:
• determining (300) the loss angle of the impedance over the frequency range;
• comparing (400) the frequency-dependent loss angle with at least one predetermined frequency-dependent loss angle of a known fatigue state of the elastomer body (20); and
• determining (500) a degree of fatigue of the elastomer body (20) from the result of the comparison (400).

2. The method according to claim 1,
wherein the frequency-dependent loss angle is compared with a plurality of predetermined frequency-dependent loss angles corresponding to different known fatigue states of the elastomer body (20).

3. The method according to claim 1 or 2,
wherein the frequency range is between 10 kHz and 10 MHz.

4. The method according to any one of claims 1 to 3,
wherein the capacitive sensor element (12) comprises a plurality of first fingers (12a) and a corresponding plurality of second fingers (12b), arranged in an interdigitated comb-like fashion.

5. The method according to claim 4,
where the webs (12a, 12b) are arranged on a carrier element (11), preferably by printing. are attached to a support element, preferably by pressing

6. The method according to claim 5,
wherein the carrier element (11) is materially bonded, preferably by adhesive bonding, to the elastomer body (20) of the elastomer product (2) on the side opposite the fingers (12a, 12b).

7. A fatigue measurement system (1) for elastomer products (2),
comprising a fatigue sensor (10) with at least one capacitive sensor element (12), configured to be arranged on a surface of an elastomer body (20) of the elastomer product (2) such that the electric field lines (B) of the capacitive sensor element (12) can penetrate into the elastomer body (20), comprising a spectrometer (13) configured to stimulate the capacitive sensor element (12), preferably wirelessly, wherein stimulation may alternatively be performed by a transmitting unit, and wherein the spectrometer (13) is configured to detect both the real part and the imaginary part of the impedance of the capacitive sensor element (12) over a frequency range,
**characterized in that,** the spectrometer (13) is further configured to determine the loss angle of the impedance over the frequency range, or alternatively the computing unit (14) is configured to determine the loss angle of the impedance over the frequency range, and comprising a computing unit (14) configured to compare the frequency-dependent loss angle with at least one predetermined frequency-dependent loss angle of a known fatigue state of the elastomer body (20) and to determine a degree of fatigue of the elastomer body (20) from the comparison result.

8. The fatigue measurement system (1) according to claim 7,
wherein the computing unit (14) is configured to compare the loss angle with a plurality of predetermined frequency-dependent loss angles corresponding to different known fatigue states of the elastomer body (20).

9. The fatigue measurement system according to claim 7 or 8,
wherein the frequency range is between 10 kHz and 10 MHz.

10. The fatigue measurement system according to any one of claims 7 to 9,
comprising a memory unit (15) configured to store the predetermined frequency-dependent impedance of a known fatigue state of the elastomer body (20), preferably a plurality of predetermined frequency-dependent impedances corresponding to different known fatigue states of the elastomer body (20), and to make them available to the computing unit (14).

11. The fatigue measurement system according to any one of claims 7 to 10,
wherein the capacitive sensor element (12) comprises a plurality of first fingers (12a) and a corresponding plurality of second fingers (12b), arranged in an interdigitated comb-like fashion.

12. The fatigue measurement system according to claim 11,
wherein the fingers (12a, 12b) are arranged on a carrier element (11), preferably by printing.

13. The fatigue measurement system according to claim 12,
wherein the carrier element (11) is materially bonded, preferably by adhesive bonding, to the elastomer body (20) of the elastomer product (2) on the side opposite the fingers (12a, 12b).

## Revendications

1. Procédé de mesure de la fatigue d'un produit en élastomère (2),
dans lequel un capteur de fatigue (10) ayant au moins un élément capteur capacitif (12) est disposé sur une surface d'un corps en élastomère (20) du produit en élastomère (2) de telle sorte que les lignes de champ électrique (B) de l'élément capteur capacitif (12) peuvent pénétrer dans le corps en élastomère (20),
comprenant au moins les étapes suivantes :
• excitation (100) de l'élément capteur capacitif (12), de préférence sans fil ;
• détection (200) à la fois de la partie réelle et de la partie imaginaire de l'impédance de l'élément capteur capacitif (12) sur une plage de fréquences ;
**caractérisé par** les étapes suivantes :
• détermination (300) de l'angle de perte de l'impédance sur la plage de fréquences ;
• comparaison (400) de l'angle de perte dépendant de la fréquence avec au moins un angle de perte dépendant de la fréquence prédéterminé d'un état de fatigue connu du corps en élastomère (20) ; et
• détermination (500) d'un degré de fatigue du corps en élastomère (20) à partir du résultat de la comparaison (400).

2. Procédé selon la revendication 1,
dans lequel l'angle de perte dépendant de la fréquence est comparé à une pluralité d'angles de perte dépendant de la fréquence prédéterminés correspondant à différents états de fatigue connus du corps en élastomère (20).

3. Procédé selon la revendication 1 ou 2,
dans lequel la plage de fréquences est comprise entre 10 kHz et 10 MHz.

4. Procédé selon l'une quelconque des revendications 1 à 3,
dans lequel l'élément capteur capacitif (12) comprend une pluralité de premiers doigts (12a) et une pluralité correspondante de seconds doigts (12b), disposés de façon interdigitée en forme de peigne.

5. Procédé selon la revendication 4,
dans lequel les doigts (12a, 12b) sont disposés sur un élément support (11), de préférence par impression.

6. Procédé selon la revendication 5,
dans lequel l'élément support (11) est lié par liaison de matière, de préférence par collage, au corps en élastomère (20) du produit en élastomère (2) sur le côté opposé aux doigts (12a, 12b).

7. Système de mesure de fatigue (1) pour des produits en élastomère (2),
comprenant un capteur de fatigue (10) avec au moins un élément capteur capacitif (12), configuré pour être disposé sur une surface d'un corps en élastomère (20) du produit en élastomère (2) de telle sorte que les lignes de champ électrique (B) de l'élément capteur capacitif (12) peuvent pénétrer dans le corps en élastomère (20), comprenant un spectromètre (13) configuré pour exciter l'élément capteur capacitif (12), de préférence sans fil, dans lequel l'excitation peut en variante être effectuée par une unité d'émission, et dans lequel le spectromètre (13) est configuré pour détecter à la fois la partie réelle et la partie imaginaire de l'impédance de l'élément capteur capacitif (12) sur une plage de fréquences,
**caractérisé en ce que**
le spectromètre (13) est en outre configuré pour déterminer l'angle de perte de l'impédance sur la plage de fréquences, ou en variante l'unité de calcul (14) est configurée pour déterminer l'angle de perte de l'impédance sur la plage de fréquences,
et comprenant une unité de calcul (14) configurée pour comparer l'angle de perte dépendant de la fréquence avec au moins un angle de perte dépendant de la fréquence prédéterminé d'un état de fatigue connu du corps en élastomère (20) et pour déterminer un degré de fatigue du corps en élastomère (20) à partir du résultat de la comparaison.

8. Système de mesure de fatigue selon la revendication 7,
dans lequel l'unité de calcul (14) est configurée pour comparer l'angle de perte à une pluralité d'angles de perte dépendant de la fréquence prédéterminés correspondant à différents états de fatigue connus du corps en élastomère (20).

9. Système de mesure de fatigue selon la revendication 7 ou 8,
dans lequel la plage de fréquences est comprise entre 10 kHz et 10 MHz.

10. Système de mesure de fatigue selon l'une quelconque des revendications 7 à 9, comprenant une unité de mémoire (15) configurée pour stocker l'impédance dépendant de la fréquence prédéterminée d'un état de fatigue connu du corps en élastomère (20), de préférence une pluralité d'impédances dépendant de la fréquence prédéterminées correspondant à différents états de fatigue connus du corps en élastomère (20), et pour les mettre à disposition de l'unité de calcul (14).

11. Système de mesure de fatigue selon l'une quelconque des revendications 7 à 10, dans lequel l'élément capteur capacitif (12) comprend une pluralité de premiers doigts (12a) et une pluralité correspondante de seconds doigts (12b), disposés de façon interdigitée en forme de peigne.

12. Système de mesure de fatigue selon la revendication 11,
dans lequel les doigts (12a, 12b) sont disposés sur un élément support (11), de préférence par impression.

13. Système de mesure de fatigue selon la revendication 12,
dans lequel l'élément support (11) est lié par liaison de matière, de préférence par collage, au corps en élastomère (20) du produit en élastomère (2) sur le côté opposé aux doigts (12a, 12b).
